# EUROPEAN PATENT SPECIFICATION

(11) **EP 2 651 465 B1**
(45) Date of publication and mention of the grant of the patent: **23.01.2019**
(21) Application number: 11849703.1
(22) Date of filing: 19.12.2011
(51) Int. Cl.: A61L 27/38, C12N 5/0775

(54) **ARTHROSCOPY METHOD**
ARTHROSKOPIEVERFAHREN
PROCÉDÉ D'ARTHROSCOPIE

(30) Priority: 17.12.2010 AU 2010905535
(43) Date of publication of application: 23.10.2013
(73) Proprietor: Cell Ideas Pty Ltd, Pymble, NSW 2073 (AU)
(72) Inventor: VESEY, Graham, Hornsby NSW 2077 (AU)
(74) Representative: Lasar, Andrea Gisela
(86) International application number: PCT/AU2011/001639
(87) International publication number: WO 2012/079132

(56) References cited:
- EP-A1- 1 908 820
- WO-A1-97/26326
- WO-A1-2010/020005
- WO-A1-2010/020005
- WO-A2-2005/042730
- WO-A2-2006/009452
- US-A1- 2006 051 865
- US-A1- 2009 060 884
- "ADICELL(TM): STEM CELL PROCEDURE FOR DOGS WITH ARTHRITIS", REGENEUS ANIMAL HEALTH, 28 July 2009 (2009-07-28), XP055118278,
- ULRICH NÖTH ET AL: "Technology Insight: adult mesenchymal stem cells for osteoarthritis therapy", NATURE CLINICAL PRACTICE RHEUMATOLOGY, 13 May 2008 (2008-05-13), XP055158149, ISSN: 1745-8382, DOI: 10.1038/ncprheum0816
- 'ADICELLTM: STEM CELL PROCEDURE FOR DOGS WITH ARTHRITIS' REGENEUS ANIMAL HEALTH, [Online] 28 July 2009, XP055118278 Retrieved from the Internet: <URL:http://www.ozpets.com.au/articles/498/ stemcellprocedurefordoQSarthritis.shtml.> [retrieved on 2012-01-09]
- JURGENS, W.J.F.M. ET AL.: 'Freshly isolated stromal cells from the infrapatellar fat pad are suitable for a one-step surgical procedure to regenerate cartilage tissue' CYTOTHERAPY vol. 11, no. 8, 2009, pages 1052 - 1064, XP008169512
- JURGENS WJ ET AL: "Freshly isolated stromal cells from the infrapatellar fat pad are suitable for a one-step surgical procedure to regenerate cartilage tissue", CYTOTHERAPY, ISIS MEDICAL MEDIA, OXFORD, GB, vol. 11, no. 8, 1 January 2009 (2009-01-01), pages 1052-1064, XP008169512, ISSN: 1465-3249, DOI: 10.3109/14653240903219122

## Description

### Field

The present invention relates to an adipose tissue derived cell suspension comprising mesenchymal stem cells for use in a method of decreasing the risk of a subject developing osteoarthritis following arthroscopic surgery of a joint.

### Background

Prior to the introduction of arthroscopy, traditional joint surgery, known as arthrotomy, involved full surgical incision of the joint. In arthrotomy the joint is completely exposed when operated on. Due to the extreme nature of this surgery full recovery is not guaranteed, particularly with geriatric patients. Arthrotomy requires lengthy surgical time periods and due to the requirement of general anesthesia an increased risk. Arthrotomy also introduces a significant risk of postoperative complications, such as infection. Arthrotomy patients endure lengthy recovery times, including extended hospital stays, and recovery is painful. The same disadvantages have been found with arthrotomies in dogs and horses.

Arthroscopy has become the alternative surgical method to arthrotomy. The employment of arthroscopy provides patients with shorter surgical times and the ability to go home the same day after surgery. Most patients that required walking aids (e.g. crutches) generally do not need them after an arthroscopic procedure. Arthroscopy recovery time is far shorter than recovery after a comparable arthrotomy and significantly less painful. Wounds are healed considerably faster with less tissue scarring due to smaller incisions. These advantages have also been identified in relation to canine and equine surgery.

The arthroscope is a fibre-optic microscope that is inserted into a joint (commonly the knee, shoulder and ankle) to evaluate and allow treatment of a number of conditions, including repair or removal of torn cartilage, the removal of loose bodies and cysts, the repair of ligaments and tendons and washing out of infected joints.

Arthroscopic surgery is the most commonly performed orthopedic procedure in humans, with more than 4 million knee arthroscopies performed worldwide each year. Over 100,000 knee arthroscopies are performed in humans in Australia alone each year. Arthroscopic surgery is also commonly performed on dogs and horses.

In horses, more than 50 joints and conditions can be treated using arthroscopy, these include the carpus (knee), fetlock, hock and stifle joints. This technique is used to treat conditions such as navicular disease, osteochondritis dissecans (OCD) lesions, bursitis, flushing infected joints, and removal of chip fractures. It is also used in equines for the treatment of tendon sheaths and tendonitis to allow full rehabilitation of the tendon and prevent scarring.

In dogs arthroscopic surgery is used to treat OCD lesions, cruciate ligament rupture, long digital extensor tendon avulsion, shoulder instability due to tears in the contra-lateral ligaments, hip and elbow dysplasia and tendonitis.

In Australia arthroscopy was introduced into veterinary medicine in the late 1970's where it was utilized as a diagnostic and surgical technique in horses. By the mid 1980's arthroscopy became commonplace in equine diagnostics and surgery, replacing arthrotomy procedures. In equine surgery arthroscopy has become the accepted method in performing all joint surgery. However, it was not until the mid 1990's where arthroscopy was introduced into small animal medicine. The delayed introduction into small animal surgery was dependant on the development of smaller instruments. In 1997, 100 dogs were diagnosed with elbow lameness using arthroscopic technique. Today in canine surgery, arthroscopy accounts for 60% of elbow surgeries followed by 30% of all shoulder surgeries and 10% for all carpal, knee and hip surgeries.

Subjects that have undergone arthroscopy are, however, at risk of developing arthritis. For example, in the case of human athletes who have had an anterior cruciate ligament rupture treated by arthroscopic surgery, it is likely that all will develop osteoarthritis over time. In a study describing 247 patients who underwent arthroscopic partial menisectomies, 38% of the subjects were classified as having worse osteoarthritis following surgery. The same study found subjects over the age of 40 were more likely to be classified with a significantly higher rate of osteoarthritis than subjects under 40. The study concluded that menisectomy in joints using arthroscopy contributed to patients developing osteoarthritis later in life.

It has been identified that 6 years after arthroscopic surgery 48% of subjects develop mild to moderate osteoarthritis in the treated joint, and of these 36% had a moderate osteoarthritis. It has been suggested that the most significant predictors of the development of osteoarthritis in joints are chondral damage from the injury, menisectomy, tendon reconstruction and the age of the patient at the time of surgery.

Cultured stem cells have been introduced into joints after arthroscopy to improve the outcome of the surgery. In these procedures, a separate surgical procedure is required to obtain a source of stem cells, which are harvested, cultured and then injected into the joint after arthroscopic surgery has been completed.

For example, in procedures utilizing mesenchymal stem cells obtained from bone marrow aspirates, the collection of stem cells and the delivery to the joint during arthroscopy was performed on separate days. Stem cells are cultured to confluency over several weeks, then prepared for intra-articular injection and injected into damaged joints via the arthroscope. These techniques thus require multiple visits to the surgery and at least two anesthetic procedures.

While allogeneic cultured cells may be used at the time of arthroscopy, this may create an increased risk of infection or malignant cell formation.

A clinical trial on the use of stem cells in arthroscopy patients is currently targeting the human arthroscopy market to treat acute meniscal tears and stimulate cartilage regeneration. Bone marrow cells were obtained and cultured to increase stem cell numbers prior to introduction into the joint. The use of tissue culture to obtain higher stem cell numbers however may alter both the morphology and function of the stem cells which, when introduced back into the joint, may have potential for malignancy. In this trial subjects undergo separate procedures on different days for obtaining a source of stem cells and for the arthroscopic re-injection of the cells.

Bone marrow stimulating technique (BMST) provides an alternative method to increase stem cell numbers in a joint to aid joint repair. BMST is an arthroscopic procedure where multiple perforations (aka micro-fractures) are made in the subchondral bone. The rationale behind BMST is to mobilize mesenchymal stem cells residing in the bone marrow so that they migrate to the joint lesion where they respond to growth factors to stimulate differentiation of fibro-cartilaginous tissue. In cases where joint debris and degenerative cartilage is arthroscopically removed prior to bone microfracture, a significant improvement has been observed in the joint.

A limitation to the use of BMST is that awl micro-fractures of the subchondral bone can cause loose bone fragments to occur in the joint. It has also been reported that the use of an awl micro-fracture technique causes acute fracture of the bone, bone compaction, which reduces the aggregation of mesenchymal stem cells to the damaged area, and high levels of osteocyte mortality.

Jurgens et al. (2009) Cytotherapy 11(8): 1052-1064 discloses that osteoarthritic defects may be treated with stromal vascular fraction cells from fat pads in a single surgical procedure.

Accordingly there is a need for alternative techniques to assist in the recovery of a subject following arthroscopy surgery, and which reduce the length of time of the procedures and/or the number of procedures required for the subject to be treated.

### Summary of the Invention

The scope of the invention is defined by the claims. Any references in the description to methods of treatment refer to the products of the present invention for use in a method for treatment. The present inventors have identified that by using autologous adipose-tissue derived stem cells present in a cell population generated by the digestion of adipose tissue from the subject, it is possible to rapidly prepare a cell suspension which comprises mesenchymal stem cells without using tissue culture and to administer this cell suspension to a joint following arthroscopic surgery within only a few hours of carrying out the arthroscopic surgery. This approach allows a subject to be treated within a single visit to the surgery and may reduce the need for multiple rounds of invasive surgical and anesthetic procedures. In addition, by using a cell suspension comprising a mixed cell population obtained from adipose tissue which comprises both mesenchymal stem cells and adipocytes, the suspension may offer not only therapeutic benefits derived from the stem cells but also from the adipocytes.

Accordingly, provided herein is an adipose tissue derived cell suspension comprising mesenchymal stem cells for use in a method of decreasing the risk of a subject developing osteoarthritis following arthroscopic surgery of a joint, wherein the method comprises:
- obtaining the cell suspension generated from isolated adipose tissue of said subject during the same surgical visit as said arthroscopy;
- administering the cell suspension to said joint during or following arthroscopic surgery of the joint within a single surgical visit.

Further provided herein is an adipose tissue derived cell suspension comprising mesenchymal stem cells and adipocytes for use in a method of decreasing the risk of a subject developing osteoarthritis following arthroscopic surgery of said joint, wherein the method comprises:
- obtaining the cell suspension from isolated adipose tissue of said subject during the same surgical visit as said arthroscopy;
- administering the cell suspension to said joint during or following arthroscopic surgery of the joint within a single surgical visit.

In certain embodiments the cell suspension is generated from adipose tissue of said subject and is administered within 3 hours of commencement of preparation of said cell suspension.

In certain embodiments the cell suspension for administration is generated from adipose tissue of said subject and is administered while the subject is continuously under general anesthesia.

Further provided herein is the use of an adipose tissue derived cell suspension comprising mesenchymal stem cells in the manufacture of a medicament for for use in a method of decreasing the risk of a subject developing osteoarthritis following arthroscopic surgery of a joint, wherein the method comprises:
- obtaining the cell suspension from adipose tissue of said subject during the same surgical visit as said arthroscopy;
- administering the cell suspension to said joint during or following arthroscopic surgery of the joint within a single surgical visit.

In certain embodiments the cell suspension comprises mesenchymal stem cells and adipocytes.

In certain embodiments the cell suspension is generated from adipose tissue of said subject and is administered within 3 hours of commencement of preparation of said cell suspension.

In certain embodiments the cell suspension for administration is generated from adipose tissue of said subject and is administered while the subject is continuously under general anesthesia.

### Detailed Description

In certain embodiments the subject is a human subject. In certain embodiments the subject is a non-human subject. The subject may be mature or juvenile. In certain embodiments the subject is a companion animal, such as a canine or a feline domestic animal, or a working animal. In other embodiments the subject is a farm animal, stud animal, or racing animal such as equines (including horses, donkeys, asses), bovines (including cattle and buffaloes), ovines, caprines, porcines, and camelids (including camels, llamas, alpacas and the like). In other embodiments the subject is a research animal, such as a rodent. In other embodiments the subject is a zoo animal, such as a member of the family Felidae, a member of the family Canidae, a member of the order Rodentia, or a member of the one of the orders of Cetacea, Perissodactyla, Artiodactyla, Tubulidentata, Hyracoidea, Sirenia, or Proboscidea.

As used herein, the term "isolated adipose tissue" is intended to encompass adipose tissue which has been removed from the body of the subject and which is no longer connected to the subject's circulation. It will be understood that "adipose tissue" may contain tissues and structures other than merely adipose, including but not necessarily limited to connective tissue and blood vessels.

The adipose tissue originates from the same individual subject in which the cell suspension comprising adipose tissue-derived mesenchymal stem cells will be administered.

The adipose tissue may originate from any source in the body which is accessible. Subcutaneous fat, for example, is readily accessible with only superficial wounding, or by using keyhole surgery techniques. Subcutaneous adipose tissue may be collected using liposuction techniques. The adipose tissue may comprise "white" adipose tissue and/or "brown" adipose tissue. In particular embodiments the adipose tissue comprises white adipose tissue only. The adipose tissue is preferably subcutaneous adipose tissue, although in certain embodiments visceral adipose tissue may be used.

The preparation of the adipose tissue-derived cell suspension may be performed in the same facility as the surgery is performed. The preparation of the adipose tissue-derived cell suspension may be performed in the same room as the surgery is performed or may be performed, in whole or in part, in a separate room of the same facility in which the arthroscopic surgery is performed.

The adipose tissue may be rinsed with a tissue culture medium or buffered isotonic solution to remove adherent red blood cells, and may be trimmed or coarsely processed to remove large blood vessels or connective tissue elements prior to generating an adipose tissue-derived cell suspension.

The term "adipose tissue-derived cell suspension" as used herein encompasses isolated cells prepared from adipose tissue or small aggregates or pieces of adipose tissue, or a mixture of two or more of isolated cells, small aggregates and pieces of adipose tissue.

The cell suspension may be obtained by mechanically dissociating adipose tissue using techniques which are readily available in the art. Any suitable method for the mechanical dissociation of adipose tissue may be used, for example by mincing adipose tissue with blades, or with scissors, or by forcing adipose tissue through screens or meshes with a pore size sufficient to break the tissue into isolated cells and/or small pieces of adipose tissue. A combination of suitable techniques may be used. Small aggregates of adipose tissue may form when dissociated adipose-derived cells reassociate into larger assemblies, for example on standing in a medium. Small pieces or aggregates of adipose tissue may be less than ten millimetres in maximum diameter, less than five millimetres in maximum diameter, less than one millimetre in maximum diameter, less than 500 µm in maximum diameter or less than 250 µm in maximum diameter. In certain embodiments, a mechanical dissociation technique is used without using one or more proteolytic enzymes. The techniques employed in these embodiments may be used to rapidly generate an adipose tissue-derived cell suspension.

The adipose tissue-derived cell suspension may be filtered through a mesh or screen to remove cell aggregates or tissue pieces which are greater than the mesh or screen pore size.

In certain embodiments, proteolytic enzymes are used to promote the dissociation of adipose tissue into an adipose tissue-derived cell suspension. Enzymes which are suitable for such a use are well known in the art, and include but are not limited to trypsin, and collagenase. It is usual to remove and/or otherwise inactivate the proteolytic enzymes before using the adipose tissue-derived cell suspension, as these enzymes may not be compatible with a desired *in vivo* use of the cell suspension. In certain embodiments, proteolytic enzymes in combination with techniques for the mechanical dissociation of adipose tissue are used to generate an adipose tissue-derived cell suspension.

The cell suspension may be suspended in a medium. The medium may be added to the adipose tissue before, during or after the dissociation of the adipose tissue. The medium may be an isotonic medium, such as a saline solution. The medium may be an isotonic buffered solution, such as a phosphate or a HEPES buffered saline or DMEM, which is capable of maintaining adipose tissue cell survival for at least one hour. The medium may be a serum free medium. In certain embodiments the medium may comprise autologous serum or serum components which support or extend adipose tissue cell survival in the cell suspension.

In a further embodiment the cell suspension is not suspended in a medium, but instead the cells are suspended in liquid which is formed during the dissociation of the tissue.

In certain embodiments the preparation of an adipose tissue-derived cell suspension comprises a centrifugation step. The centrifugation of isolated cells or small aggregates or pieces of adipose tissue suspended in a liquid, such as a medium, is at approximately 500 g for 10 minutes, or for sufficient time and at a sufficient g-force to generate a cell pellet which comprises adipose-derived non-adipocyte cells, including mesenchymal stem cells, above which is a layer of medium, floating above which in turn is a layer which comprises adipocytes, and floating at the top is a layer of lipid which is derived from ruptured adipocytes. Following centrifugation, in certain embodiments the lipid layer and the medium layer will be discarded and the retained cells are mixed, leaving an adipose tissue-derived cell suspension which comprises adipocytes and adipose-derived non-adipocyte cells. Following centrifugation, in certain embodiments, all of the layers are discarded except the non-adipocyte cell layer which comprises mesenchymal stem cells.

In certain embodiments multiple centrifugation steps may be used, for example to provide additional cell separation steps.

In other embodiments, the preparation of an adipose tissue-derived cell suspension does not include a centrifugation step.

In certain embodiments the adipose tissue-derived cell suspension comprises adipocytes. In particular embodiments the adipocytes comprise viable adipocytes. In particular embodiments, the adipocytes retain detectable quantities of lipid in their cytoplasm, and may be separated from adipose-derived non-adipocyte cells on the basis of the different density provided by the lipid. Lipid may be detectable using light microscopy techniques, including phase contrast microscopy, or by staining a sample of cells with a lipophilic dye such as Oil Red O. Adipocytes which retain lipid in their cytoplasm are considerably more fragile than other adipose-derived cells and accordingly, where viable adipocytes are required, techniques for dissociating tissue which damage or render non-viable a large proportion of the adipocytes should be avoided. The ultrasonic dissociation of adipose tissue or techniques in which adipose tissue is vigorously shaken, for example, are unlikely to provide a cell suspension which contains large numbers of viable adipocytes. The viability of adipocytes may readily be determined using readily available techniques, such as the LIVE/DEAD cell viability assays (Molecular Probes).

The number of cells present in the cell suspension may be adjusted prior to administration; however the concentration of cells generated from isolated adipose tissue using the techniques described herein was shown to be both tolerated and therapeutically effective.

Administration of the cell suspension may be made by any suitable means which allows the introduction of cells into the synovial space or into the joint space between damaged joint cartilage. For example, administration of the cell suspension may be made using a port or catheter attached to the arthroscope or by syringe with a needle inserted into the joint, or by micro pump or peristaltic pump.

Typically, administration of the cell suspension is by a member of the same team that performs the arthroscopy although it may be performed by a different individual.

The cell suspension may be administered to the joint following completion of the arthroscopic surgery. In this context it will be understood that administration of the cell suspension following arthroscopic surgery means that the primary intention of the practitioner in performing arthroscopy, such as internal visualization of the state of the joint or surgical intervention within the joint, such as for repair or treatment of the joint, has been completed or substantially completed. The cell suspension may be administered during the arthroscopy procedure in which case the arthroscope may still be in place in the joint. In certain embodiments the cell suspension is administered immediately after the arthroscopy is completed. In certain embodiments the cell suspension is administered within 5 minutes after arthroscopy, or within 10 minutes after arthroscopy, or within 15 minutes after arthroscopy, or within 20 minutes after arthroscopy, or within 25 minutes after arthroscopy, or within 30 minutes after arthroscopy, or within 60 minutes after arthroscopy.

One option is to administer the cell suspension after the surgery using a small bore egress fluid hypodermic needle which may be placed in the joint during the arthroscopy procedure to act as an outlet for saline which is infused into the joint during surgery to keep the joint expanded. Using this approach, the cell suspension may be administered after the completion of arthroscopy and the removal of the arthroscope. A suture may be used to close the hole that the arthroscope was inserted into and the suspension is then administered via the egress needle. Where administration is made to a joint following arthroscopy typically the synovial fluid of the joint has been flushed away during the arthroscopic procedure.

The volume of cell suspension to be administered to the joint will depend on the type of joint, and the species and size of the subject. Where administration is by injection into the synovial fluid of a joint the volume of cell suspension administered may be constrained by the volume of the synovial fluid which may be held at the joint, which in turn depends on the type of joint and the species and size of the subject. A volume of from 0.5 ml to 10 ml is typical for administration.

In certain embodiments the cell suspension is suspended in a biologically tolerated solution which increases the viscosity of the cell suspension, such as a collagen, alginate, fibrin, hyaline, plasma or Matrigel™ solution, prior to administration. In certain embodiments the cell suspension may be administered mixed with or in concert with hyaluronin or other agents to replace the synovial fluid.

Administration of the cell suspension into the joint may be made through any suitable method, for example by utilizing a port in the arthroscope, or by needle guided by the arthroscope, by ultrasound, by x-ray imaging or guided by touch.

Typically joint disorders in humans involve at least one joint in one or both hips, knees, ankles, elbows, shoulders, wrists, the metacarpo-phalangeal articulations or the phalangeal articulations, the metatarso-tarsal articulations or the tarsal articulations or between two or more vertebrae. For veterinary joint-related disorders the corresponding joints are involved in mammalian animals, and these include the stifle and hock joints.

Administration of the cell suspension may be made to the joint cavity which is usually occupied by synovial fluid. Administration of the cell suspension may be intra-articular.

The invention may be used for any arthroscopic procedure where the arthroscopic procedure increases the risk of subsequently developing osteoarthritis.

One aim of the invention is to provide a rapid method by which a cell suspension comprising mesenchymal stem cells may be generated, and thus enable the surgical team to have sufficient time to produce a cell suspension comprising mesenchymal cells from the subject, to carry out an arthroscopic surgical procedure to a joint and to administer the cell suspension to the joint within a single visit to the surgery, and preferably without requiring supplemental anesthesia to the anesthesia required for the arthroscopic procedure.

The time required to isolate a source of adipose tissue from the subject and to generate an adipose tissue derived cell suspension which comprises mesenchymal stem cells suitable for administration to the joint is typically in the range of from 1 hour to 3 hours, including the steps of obtaining an adipose tissue sample, rinsing the sample and mincing it with blades or scissors, digesting the minced sample in a proteolytic or collagen-degrading enzyme, rinsing and centrifuging the cell suspension, and preparing a sample of the cell suspension for administration. In certain embodiments the time required to isolate adipose tissue from the subject and to generate a cell suspension suitable for administration to a joint is between 1 hour and 3 hours. In certain embodiments the time required to isolate adipose tissue from the subject and to generate a cell suspension suitable for administration to a joint is between 1 hour and 2 hours. In certain embodiments the time required to isolate adipose tissue from the subject and to generate a cell suspension suitable for administration to a joint is between 1 hour and 1.5 hours.

Typically the isolation of the adipose tissue will commence before the commencement of the arthroscopic procedure to allow maximal time for the cell suspension to be prepared before the arthroscopic procedure is completed. In certain embodiments the isolation of adipose tissue will commence after the subject has been sedated or received local or regional anesthesia or has been subject to general anesthesia. In certain embodiments the isolation of the adipose tissue will commence immediately before (ie within 30 minutes to one hour before) commencing the arthroscopic procedure. In certain embodiments, such as for example where a horse will be subject to an arthroscopic procedure, the isolation of adipose tissue may take place between 2 and 6 hours before commencement of the arthroscopic procedure. In certain embodiments the isolation of adipose tissue may take place between 2 and 5 hours before commencement of the arthroscopic procedure. In certain embodiments the isolation of adipose tissue may take place between 2 and 4 hours before commencement of the arthroscopic procedure.

In certain embodiments the isolation of adipose tissue is carried out while the subject is under general anesthesia. In these embodiments, preferably the arthroscopic procedure is also carried out whilst the subject is under the same round of anesthesia.

In certain embodiments the arthroscopic procedure is carried out while the subject is under regional; or local anesthesia or local anesthesia. In these cases the isolation of adipose tissue will typically be carried out under regional or local anesthesia of the area from which the adipose tissue is to be obtained or isolated. In certain embodiments one or both of the isolation of the adipose tissue and the arthroscopic surgery may be carried out under intravenous sedation, which may be in conjunction with local or regional anesthesia.

In certain embodiments the isolation of adipose tissue is carried out while the subject has been provided with a local anesthetic or a spinal block.

Typically the generation of a cell suspension from the isolated adipose tissue and the arthroscopic surgery are carried out by different operators, to allow the complete procedure to complete in as short a time period as practicable.

Once the cell suspension is prepared from the isolated adipose tissue, it may be stored in sterile medium or a biologically compatible isotonic solution in a 37°C incubator or water bath for at least one hour, if required, to allow time for the arthroscopic procedure to finish. Alternatively, where the arthroscopic procedure is expected to take longer than the preparation of the cell suspension, the commencement of the preparation of the cell suspension can be delayed so that the cell suspension is ready shortly before the completion of the arthroscopic procedure.

One aim of the invention is to permit the use of an adipose tissue-derived cell suspension comprising mesenchymal stem cells, optionally also comprising adipocytes, which has been freshly prepared or generated from the subject's adipose tissue. A freshly prepared cell suspension is one that has been prepared on the same day as it is to be administered to the subject. In one embodiment the preparation of the cell suspension is completed within 4 hours prior to administration to the subject. In one embodiment the preparation of the cell suspension is completed within 3 hours prior to administration to the subject. In one embodiment the preparation of the cell suspension is completed within 2 hours prior to administration to the subject. In one embodiment the preparation of the cell suspension is completed within 1 hour prior to administration to the subject. In one embodiment the preparation of the cell suspension is completed within 30 minutes prior to administration to the subject. In one embodiment the preparation of the cell suspension is completed within 20 minutes prior to administration to the subject. In one embodiment the preparation of the cell suspension is completed within 10 minutes prior to administration to the subject. In one embodiment the preparation of the cell suspension is completed within 5 minutes prior to administration to the subject. In one embodiment the preparation of the cell suspension is completed immediately before administration to the subject.

Preparation of the cell suspension within the same surgical visit by the subject allows the use of a fresh cell suspension. Use of a fresh cell suspension may offer advantages over the use of a cell suspension which is not freshly prepared. For example, the use of a fresh cell suspension compared to a cell suspension comprising cultured cells reduces the risk of morphological and or functional changes in the cells, whose changes which may have the potential for malignancy. The use of freshly prepared cell suspension also reduces the requirement for storage of the cell suspension prior to administration, thereby offering the potential for retention of greater cell viability, for example of the mesenchymal stem cells and, when optionally present, the adipose cells or adipocytes. The use of freshly prepared adipose tissue-derived cell suspension also reduces the potential for contamination of the cell suspension, thereby reducing the need for inclusion of antibiotics during the preparation of the cell suspension or in the cell suspension itself. The use of freshly prepared adipose tissue-derived cell suspension also offers advantages to the subject being treated as described herein.

In certain embodiments the isolation of adipose tissue from the subject and the generation of a cell suspension and the administration of the cell suspension following arthroscopic surgery is carried out within a single surgical visit. As used herein the term "within a single surgical visit" is intended to mean the period over which the subject is continuously sedated and/or anesthetized, or the period over which the subject is subject to multiple rounds of sedation and/or general or local anesthesia while the subject remains in the surgery or recovery room. The term "within a single surgical visit" does not encompass surgery which comprises one or multiple rounds of sedation and/or anesthesia over a period of more than 12 hours.

In certain embodiments the methods described herein provide a method of promoting recovery from an arthroscopic procedure. Promoting recovery from an arthroscopic procedure is intended to encompass hastening the rate of recovery, or improving the outcome of an arthroscopic procedure, or reducing or preventing complication(s) arising from arthroscopic surgery, such as developing osteoarthritis, or any combination of those effects, when compared to a procedure in which mesenchymal stem cells, and optionally also adipocytes, are not administered.

The invention provides an adipose tissue derived cell suspension comprising mesenchymal stem cells for use in a method of decreasing the risk of a subject developing osteoarthritis following arthroscopic surgery, which in alternative language includes preventing or reducing the severity of osteoarthritis which may develop following arthroscopy.

In certain embodiments, administration of the adipose tissue-derived cell suspension comprising mesenchymal stem cells, optionally also comprising adipocytes, may in conjunction with the arthroscopic surgery, or as an adjunct thereto, promote improvement in or reduce or prevent deterioration of the underlying joint condition which is the subject of the arthroscopy. The joint may be any joint of the body which is susceptible to arthroscopy.

The invention may be used in conjunction with, such as following or during, any arthroscopic procedure performed on a joint. Generally described, the arthroscopy may be for visualisation of the joint, such as to evaluate the condition of the joint, for example to assist diagnosis, or may be for treatment of a joint, or both. Arthroscopy is commonly used in treatment, including repair or removal of torn cartilage, the removal of loose bodies and cysts, the repair of ligaments and tendons and washing out of infected joints. In some embodiments the arthroscopy includes surgical treatment of a surgically treatable condition. In some embodiments the arthroscopy does not include surgical treatment of a surgically treatable condition.

In certain embodiments the joint may be a knee, shoulder, hip, ankle, elbow or wrist.

In certain embodiments the joint condition, which may alternatively be referred to as a joint disorder, is any detrimental condition of a joint which is amenable to arthroscopy. In certain embodiments the joint condition may be lameness or pain, including lameness or pain for which a clinically diagnosable medical or veterinary condition other than the lameness or pain may not be apparent. Typically in such embodiments the arthroscopy does not include surgical treatment as no surgically treatable condition is identified.

In certain embodiments, typically for arthroscopy of the knee, the arthroscopic surgery is removal of a portion of the meniscus cartilage, commonly also referred to as treatment of a torn cartilage or meniscus surgery (menisectomy), meniscus repair, meniscus transplant, treatment of a cruciate ligament, such as the anterior cruciate ligament (ACL), in which treatment is typically repair of the ligament, treatment of plica syndrome, typically by plica excision or plica resection, lateral release to loosen ligaments of the kneecap, treatment of damaged cartilage, such as by bone marrow stimulating technique (BMST) as described above, for example microfracture to stimulate growth of new cartilage, cartilage transfer such as chondrocyte implantation, mosaicplasty and osteochondral autograft transfer system (OATS). In certain embodiments the knee joint condition is a condition for which any of the arthroscopic treatments mentioned herein is appropriate.

In certain embodiments the shoulder joint condition may be unexplained shoulder pain, a rotator cuff tear, shoulder instability including labral tear such as Bankart lesions and SLAP (superior labrum from anterior to posterior) tear, impingement syndrome, such as may be treated by arthroscopic subacromial decompression, biceps tendonitis, frozen shoulder, acromioclavicular (AC) joint arthritis, shoulder bursitis.

In certain embodiments the hip joint condition may be a labral tear, loose bodies such as pieces of cartilage or bone, snapping hip syndrome, cartilage damage such as focal cartilage damage, early arthritis, bone spurs.

In certain embodiments the ankle joint condition may be cartilage damage, in which arthroscopy may be to restore cartilage to the surface of the joint, such as by microfracture, cartilage transfer or chondrocyte implantation, a joint condition associated with the presence of bone spurs, such as anterior ankle impingement syndrome, the presence of loose debris, or the presence of scar tissue, or posterior ankle pain.

In certain embodiments the elbow joint condition is the presence of loose debris, such as a result of arthritis or injury, which may be removed by arthroscopy, bone spurs, thrower's elbow, elbow arthrofibrosis, such as may be treated by arthroscopic loosening of the joint capsule, specific cartilage problem, such as osteochondritis dessecans (OCD), tennis elbow, microscopic tear of tendons.

In certain embodiments the wrist joint condition is a cartilage problem, such as damage to the triangular fibrocartilage complex (TFCC), wrist fracture involving cartilage of the wrist, ganglion cysts.

In certain embodiments the subject is a human. In certain embodiments the subject is a non-human animal. In certain embodiments the non-human animal is a dog, cat or horse.

In horses, more than 50 joints and conditions can be treated using arthroscopy, including the carpus (knee), fetlock, hock and stifle joints, all of which are contemplated for the present invention. In certain embodiments the joint condition is navicular disease, osteochondritis dissecans (OCD) lesions, bursitis, flushing infection of the joint, the presence of chip fractures or other debris, tendon sheath damage or deterioration, tendonitis, scarring.

In dogs, arthroscopy is commonly used in treatment of joint conditions including the elbow, shoulder, carpal, knee and hip, all of which are contemplated for the present invention. In certain embodiments the joint condition is OCD lesions, cruciate ligament rupture, long digital extensor tendon avulsion, shoulder instability due to tears in the contra-lateral ligaments, hip and elbow dysplasia and tendonitis.

In the context of this specification, the term "comprising" means including but not necessarily solely including. Furthermore, variations of the word "comprising" such as "comprise" and "comprises" have correspondingly varied meanings.

Throughout this specification, reference to "a" or "one" element does not exclude the plural, unless context determines otherwise.

The examples which follow are intended to serve to illustrate this invention and should not be construed as limiting the general nature of the disclosure of the description throughout this specification.

### Examples

### Example 1. Intra-articular administration of adipose derived stem cells to a dog

A four year old Australian cattle dog with severe lameness in the right front leg was treated by arthroscopic surgery and an intra-articular injection of adipose derived cells. Radiographs of the front right leg of the dog prior to arthroscopic surgery did not identify the cause of the lameness.

In the day of arthroscopic surgery the dog was given a general anesthetic and approximately 10 g of adipose tissue was collected by excision from the groin.

Immediately after obtaining an adipose tissue sample, an arthroscopy procedure was performed on the right elbow whilst the adipose tissue was being processed. The arthroscopy revealed some scoring of the cartilage but no cartilage or bone flaps.

The isolated adipose tissue was processed to generate a cell population. The adipose tissue was rinsed with sterile saline, minced finely using scissors and mixed with 20 ml of Dulbecco's Modified Eagle's Medium (DMEM, Sigma). Collagenase (Sigma) was added to achieve a final concentration of 0.05% and the sample was incubated at 37°C for 90 minutes. During the incubation the sample was gently inverted by hand every 15 minutes.

Following collagenase treatment the sample was aseptically filtered through a stainless steel mesh (300 µm pore size) to remove large pieces of undigested tissue, and the cell preparation which passed through the mesh was transferred to a 50 ml centrifuge tube and centrifuged at 500g for 15 minutes at 4°C.

Four distinct layers were visible within the sample following centrifugation: a small (2 mm thick) layer of free lipid on the surface, below which was a white layer of adipocytes 10 mm thick and then a large clear layer of liquid which largely comprised DMEM and then at the bottom of the centrifuge tube a pellet of adipose-derived non-adipocyte cells. The small layer of lipid was carefully removed with a pasteur pipette. A fresh pasteur pipette was then carefully inserted through the adipocytes and the clear DMEM was removed without disturbing the floating adipocytes or the pelleted cells. This resulted in a sample that contained only the floating adipocytes and the pelleted cells. The floating adipocytes and the pelleted cells were gently mixed with a pasteur pipette and transferred to a 15 ml centrifuge tube.

The cells were then washed in DMEM to remove collagenase. DMEM was added to a final volume of 14 ml and the sample centrifuged at 500g for 10 minutes. This resulted in three distinct layers: floating adipocytes, DMEM and pelleted adipose-derived non-adipocyte cells. The DMEM was carefully removed by inserting a pasteur pipette through the adipocytes taking care not to disturb the adipocytes or the pelleted cells.

The floating and the pelleted cells were gently resuspended in 4 ml of DMEM and mixed with a pasteur pipette. The cell suspension was taken up into a sterile syringe ready for injection into the joint.

Arthroscopy did not reveal any lesions amenable to surgical treatment such as osteochondrosis dissecans or removable fracture bodies. The arthroscope was removed and the entry was closed with a single suture. The cells were injected through the egress needle into the joint space. The complete procedure was finished within 2 hours of the initial administration of anesthetic and within a single round of anesthesia.

The dog was examined at two weeks post treatment and the improvement in lameness was dramatic. Such an improvement would not be anticipated from the arthroscopy procedure, particularly as the procedure did not involve surgical treatment because no surgically treatable condition was identified. At 4 months post treatment the dog was back at work herding cattle.

### Example 2. Intra-articular administration of adipose derived stem cells to a horse

A 21 year old horse with severe lameness in the right front leg underwent arthroscopic surgery and received an intra-articular injection of adipose derived cells.

A sample of adipose tissue of approximately 20 g in wet weight was collected by excision from the left flank whilst the horse was sedated prior to arthroscopy. Whilst the adipose tissue was being processed an arthroscopy procedure was performed on the knee. Bone and cartilage fragments were flushed from the joint during the arthroscopic procedure.

The adipose tissue was rinsed with saline and then minced finely using scissors and mixed with 20 ml of Dulbecco's Modified Eagle's Medium (DMEM, Sigma). Collagenase (Sigma) was added to achieve a final concentration of 0.05% and the sample was incubated at 37°C for 60 minutes. During the incubation the sample was gently inverted by hand every 15 minutes.

Following collagenase treatment the sample was aseptically filtered through a mesh (300 µm pore size), transferred to a 50 ml centrifuge tube and centrifuged at 500g for 15 minutes at 4°C.

Four distinct layers were visible within the centrifuged sample: a small (2 mm thick) layer of free lipid on the surface, below which was a white 10 mm thick layer of adipocytes and then a large clear layer of liquid which largely comprised DMEM and then a pellet of adipose-derived non-adipocyte cells. The small layer of lipid was carefully removed with a pasteur pipette. A fresh pasteur pipette was then carefully inserted through the adipocytes and the clear DMEM was removed without disturbing the floating adipocytes or the pelleted cells. This resulted in a sample that contained only the floating adipocytes and the pelleted cells. The floating adipocytes and the pelleted cells were gently mixed with a pasteur pipette and transferred to a 15 ml centrifuge tube.

The cells were then washed in DMEM to remove collagenase. DMEM was added to a final volume of 14 ml and the sample centrifuged at 500g for 10 minutes. This resulted in three distinct layers: floating adipocytes, DMEM and pelleted adipose-derived non-adipocyte cells. The DMEM was carefully removed by inserting a pasteur pipette through the adipocytes taking care not to disturb the adipocytes or the pelleted cells.

The floating and the pelleted cells were gently resuspended in 4 ml of DMEM and mixed with a pasteur pipette. The cell suspension was taken up into a sterile syringe and injected into the knee joint immediately after arthroscopy whilst the horse was still under anesthetic.

Arthroscopy did not reveal any lesions amenable to surgical treatment such as osteochondrosis dissecans or removable fracture bodies. The arthroscope was removed and the entry was closed with a single suture. The cells were injected through the egress needle into the joint space.

Two weeks after treatment the horse was moving freely. The reduction in lameness of the subject was far greater than expected from the arthroscopy alone. Such an improvement would not be anticipated from the arthroscopy procedure, particularly as the procedure did not involve surgical treatment because no surgically treatable condition was identified.

While embodiments of the present invention have been described and illustrated herein, those of ordinary skill in the art will readily envision a variety of other means for performing the functions and/or obtaining the results and/or one or more of the advantages described herein, and each of such variations and/or modifications of the invention described herein is deemed to be within the scope of the present invention.

## Claims

1. An adipose tissue derived cell suspension comprising mesenchymal stem cells for use in a method of decreasing the risk of a subject developing osteoarthritis following arthroscopic surgery of a joint, wherein the method comprises:
- obtaining the cell suspension from isolated adipose tissue of said subject during the same surgical visit as said arthroscopy; and
- administering the cell suspension to said joint during or following arthroscopic surgery of the joint within a single surgical visit.

2. An adipose tissue derived cell suspension comprising mesenchymal stem cells and adipocytes for use in a method of decreasing the risk of a subject developing osteoarthritis following arthroscopic surgery of a joint, wherein the method comprises:
- obtaining the cell suspension from isolated adipose tissue of said subject during the same surgical visit as said arthroscopy; and
- administering said cell suspension to said joint during or following arthroscopic surgery of the joint within a single surgical visit.

3. The adipose tissue derived cell suspension comprising mesenchymal stem cells for use in a method of decreasing the risk of a subject developing osteoarthritis following arthroscopic surgery of a joint according to claim 1, wherein the method comprises:
- generating the cell suspension from isolated adipose tissue of said subject and administering the cell suspension within 3 hours of commencement of preparation of said cell suspension.

4. The adipose tissue derived cell suspension comprising mesenchymal stem cells and adipocytes for use in a method of decreasing the risk of a subject developing osteoarthritis following arthroscopic surgery of a joint according to claim 2, wherein the method comprises:
- generating the cell suspension from isolated adipose tissue of said subject and administering the cell suspension within 3 hours of commencement of preparation of said cell suspension.

5. The adipose tissue derived cell suspension comprising mesenchymal stem cells for use in a method of decreasing the risk of a subject developing osteoarthritis following arthroscopic surgery of a joint according to claim 1 or the adipose tissue derived cell suspension comprising mesenchymal stem cells and adipocytes for use in a method of decreasing the risk of a subject developing osteoarthritis following arthroscopic surgery of a joint according to claim 2, wherein the method comprises:
- generating the cell suspension from isolated adipose tissue of said subject and administering the cell suspension while the subject is continuously under general anesthesia.

6. Use of an adipose tissue derived cell suspension comprising mesenchymal stem cells in the manufacture of a medicament for use in a method of decreasing the risk of a subject developing osteoarthritis following arthroscopic surgery of a joint, wherein the method comprises:
- obtaining the cell suspension from isolated adipose tissue of said subject during the same surgical visit as said arthroscopy; and
- administering the cell suspension to said joint during or following arthroscopic surgery of the joint within a single surgical visit.

7. Use of an adipose tissue derived cell suspension comprising mesenchymal stem cells and adipocytes in the manufacture of a medicament for use in a method of decreasing the risk of a subject developing osteoarthritis following arthroscopic surgery of a joint, wherein the method comprises:
- obtaining the cell suspension from isolated adipose tissue of said subject during the same surgical visit as said arthroscopy; and
- administering the cell suspension to said joint during or following arthroscopic surgery of the joint within a single surgical visit.

8. Use of an adipose tissue derived cell suspension comprising mesenchymal stem cells in the manufacture of a medicament for use in a method of decreasing the risk of a subject developing osteoarthritis following arthroscopic surgery of a joint according to claim 6, wherein the method comprises:
- generating the cell suspension from isolated adipose tissue of said subject and administering the cell suspension within 3 hours of commencement of preparation of said cell suspension.

9. Use of an adipose tissue derived cell suspension comprising mesenchymal stem cells in the manufacture of a medicament for use in a method of decreasing the risk of a subject developing osteoarthritis following arthroscopic surgery of a joint according to claim 6, wherein the method comprises:
- generating the cell suspension for administration from isolated adipose tissue of said subject and administering the cell suspension while the subject is continuously under general anesthesia.

## Patentansprüche

1. Aus Fettgewebe stammende Zellsuspension umfassend mesenchymale Stammzellen zur Verwendung in einem Verfahren zur Verringerung des Risikos eines Subjekts, nach arthroskopischer Operation eines Gelenks Osteoarthritis zu entwickeln, wobei das Verfahren umfasst:
- Erhalten der Zellsuspension aus isoliertem Fettgewebe des Subjekts während desselben chirurgischen Eingriffs wie die Arthroskopie; und
- Verabreichen der Zellsuspension an das Gelenk während oder nach der arthroskopischen Operation des Gelenks innerhalb eines einzigen chirurgischen Eingriffs.

2. Aus Fettgewebe stammende Zellsuspension umfassend mesenchymale Stammzellen und Adipozyten zur Verwendung in einem Verfahren zur Verringerung des Risikos eines Subjekts, nach arthroskopischer Operation eines Gelenks Osteoarthritis zu entwickeln, wobei das Verfahren umfasst:
- Erhalten der Zellsuspension aus isoliertem Fettgewebe des Subjekts während desselben chirurgischen Eingriffs wie die Arthroskopie; und
- Verabreichen der Zellsuspension an das Gelenk während oder nach der arthroskopischen Operation des Gelenks innerhalb eines einzigen chirurgischen Eingriffs.

3. Die aus Fettgewebe stammende Zellsuspension umfassend mesenchymale Stammzellen zur Verwendung in einem Verfahren zur Verringerung des Risikos eines Subjekts, nach arthroskopischer Operation eines Gelenks Osteoarthritis zu entwickeln nach Anspruch 1, wobei das Verfahren umfasst:
- Erhalten der Zellsuspension aus isoliertem Fettgewebe des Subjekts und Verabreichen der Zellsuspension innerhalb von 3 Stunden nach Beginn der Herstellung der Zellsuspension.

4. Die aus Fettgewebe stammende Zellsuspension umfassend mesenchymale Stammzellen und Adipozyten zur Verwendung in einem Verfahren zur Verringerung des Risikos eines Subjekts, nach arthroskopischer Operation eines Gelenks Osteoarthritis zu entwickeln nach Anspruch 2, wobei das Verfahren umfasst:
- Erhalten der Zellsuspension aus isoliertem Fettgewebe des Subjekts und Verabreichen der Zellsuspension innerhalb von 3 Stunden nach Beginn der Herstellung der Zellsuspension.

5. Die aus Fettgewebe stammende Zellsuspension umfassend mesenchymale Stammzellen zur Verwendung in einem Verfahren zur Verringerung des Risikos eines Subjekts, nach arthroskopischer Operation eines Gelenks Osteoarthritis zu entwickeln nach Anspruch 1 oder die aus Fettgewebe stammende Zellsuspension umfassend mesenchymale Stammzellen und Adipozyten zur Verwendung in einem Verfahren zur Verringerung des Risikos eines Subjekts, nach arthroskopischer Operation eines Gelenks Osteoarthritis zu entwickeln nach Anspruch 2, wobei das Verfahren umfasst:
- Erzeugen der Zellsuspension aus isoliertem Fettgewebe des Subjekts und Verabreichen der Zellsuspension, während das Subjekt kontinuierlich unter Vollnarkose ist.

6. Verwendung einer aus Fettgewebe stammenden Zellsuspension umfassend mesenchymale Stammzellen zur Herstellung eines Medikaments zur Verwendung in einem Verfahren zur Verringerung des Risikos eines Subjekts, nach arthroskopischer Operation eines Gelenks Osteoarthritis zu entwickeln, wobei das Verfahren umfasst:
- Erhalten der Zellsuspension aus isoliertem Fettgewebe des Subjekts während desselben chirurgischen Eingriffs wie die Arthroskopie; und
- Verabreichen der Zellsuspension an das Gelenk während oder nach der arthroskopischen Operation des Gelenks innerhalb eines einzigen chirurgischen Eingriffs.

7. Verwendung einer aus Fettgewebe stammenden Zellsuspension umfassend mesenchymale Stammzellen und Adipozyten zur Herstellung eines Medikaments zur Verwendung in einem Verfahren zur Verringerung des Risikos eines Subjekts, nach arthroskopischer Operation eines Gelenks Osteoarthritis zu entwickeln, wobei das Verfahren umfasst:
- Erhalten der Zellsuspension aus isoliertem Fettgewebe des Subjekts während desselben chirurgischen Eingriffs wie die Arthroskopie; und
- Verabreichen der Zellsuspension an das Gelenk während oder nach der arthroskopischen Operation des Gelenks innerhalb eines einzigen chirurgischen Eingriffs.

8. Verwendung einer aus Fettgewebe stammenden Zellsuspension umfassend mesenchymale Stammzellen zur Herstellung eines Medikaments zur Verwendung in einem Verfahren zur Verringerung des Risikos eines Subjekts, nach arthroskopischer Operation eines Gelenks Osteoarthritis zu entwickeln nach Anspruch 6, wobei das Verfahren umfasst:
- Erzeugen der Zellsuspension aus isoliertem Fettgewebe des Subjekts und Verabreichen der Zellsuspension innerhalb von 3 Stunden nach Beginn der Herstellung der Zellsuspension.

9. Verwendung einer aus Fettgewebe stammenden Zellsuspension umfassend mesenchymale Stammzellen zur Herstellung eines Medikaments zur Verwendung in einem Verfahren zur Verringerung des Risikos eines Subjekts, nach arthroskopischer Operation eines Gelenks Osteoarthritis zu entwickeln nach Anspruch 6, wobei das Verfahren umfasst:
- Erzeugen der Zellsuspension aus isoliertem Fettgewebe des Subjekts und Verabreichen der Zellsuspension, während das Subjekt kontinuierlich unter Vollnarkose ist.

## Revendications

1. Suspension cellulaire dérivée de tissu adipeux comprenant des cellules souches mésenchymateuses destinée à être utilisée dans un procédé de diminution du risque qu'un sujet développe de l'ostéoarthrite suite à une chirurgie arthroscopique d'une articulation, sachant que le procédé comprend :
- l'obtention de la suspension cellulaire à partir de tissu adipeux isolé dudit sujet pendant la même visite chirurgicale que ladite arthroscopie ; et
- l'administration de la suspension cellulaire à ladite articulation pendant ou suite à une chirurgie arthroscopique de l'articulation au cours d'une même visite chirurgicale.

2. Suspension cellulaire dérivée de tissu adipeux comprenant des cellules souches mésenchymateuses et des adipocytes destinée à être utilisée dans un procédé de diminution du risque qu'un sujet développe de l'ostéoarthrite suite à une chirurgie arthroscopique d'une articulation, sachant que le procédé comprend :
- l'obtention de la suspension cellulaire à partir de tissu adipeux isolé dudit sujet pendant la même visite chirurgicale que ladite arthroscopie ; et
- l'administration de ladite suspension cellulaire à ladite articulation pendant ou suite à une chirurgie arthroscopique de l'articulation au cours d'une même visite chirurgicale.

3. La suspension cellulaire dérivée de tissu adipeux comprenant des cellules souches mésenchymateuses destinée à être utilisée dans un procédé de diminution du risque qu'un sujet développe de l'ostéoarthrite suite à une chirurgie arthroscopique d'une articulation selon la revendication 1, sachant que le procédé comprend :
- la génération de la suspension cellulaire à partir de tissu adipeux isolé dudit sujet et l'administration de la suspension cellulaire dans les 3 heures à compter de la préparation de ladite suspension cellulaire.

4. La suspension cellulaire dérivée de tissu adipeux comprenant des cellules souches mésenchymateuses et des adipocytes destinée à être utilisée dans un procédé de diminution du risque qu'un sujet développe de l'ostéoarthrite suite à une chirurgie arthroscopique d'une articulation selon la revendication 2, sachant que le procédé comprend :
- la génération de la suspension cellulaire à partir de tissu adipeux isolé dudit sujet et l'administration de la suspension cellulaire dans les 3 heures à compter de la préparation de ladite suspension cellulaire.

5. La suspension cellulaire dérivée de tissu adipeux comprenant des cellules souches mésenchymateuses destinée à être utilisée dans un procédé de diminution du risque qu'un sujet développe de l'ostéoarthrite suite à une chirurgie arthroscopique d'une articulation selon la revendication 1 ou la suspension cellulaire dérivée de tissu adipeux comprenant des cellules souches mésenchymateuses et des adipocytes destinée à être utilisée dans un procédé de diminution du risque qu'un sujet développe de l'ostéoarthrite suite à une chirurgie arthroscopique d'une articulation selon la revendication 2, sachant que le procédé comprend :
- la génération de la suspension cellulaire à partir de tissu adipeux isolé dudit sujet et l'administration de la suspension cellulaire pendant que le sujet est continuellement sous anesthésie générale.

6. Utilisation d'une suspension cellulaire dérivée de tissu adipeux comprenant des cellules souches mésenchymateuses dans la fabrication d'un médicament destiné à être utilisé dans un procédé de diminution du risque qu'un sujet développe de l'ostéoarthrite suite à une chirurgie arthroscopique d'une articulation, sachant que le procédé comprend :
- l'obtention de la suspension cellulaire à partir de tissu adipeux isolé dudit sujet pendant la même visite chirurgicale que ladite arthroscopie ; et
- l'administration de la suspension cellulaire à ladite articulation pendant ou suite à une chirurgie arthroscopique de l'articulation au cours d'une même visite chirurgicale.

7. Utilisation d'une suspension cellulaire dérivée de tissu adipeux comprenant des cellules souches mésenchymateuses et des adipocytes dans la fabrication d'un médicament destiné à être utilisé dans un procédé de diminution du risque qu'un sujet développe de l'ostéoarthrite suite à une chirurgie arthroscopique d'une articulation, sachant que le procédé comprend :
- l'obtention de la suspension cellulaire à partir de tissu adipeux isolé dudit sujet pendant la même visite chirurgicale que ladite arthroscopie ; et
- l'administration de la suspension cellulaire à ladite articulation pendant ou suite à une chirurgie arthroscopique de l'articulation au cours d'une même visite chirurgicale.

8. Utilisation d'une suspension cellulaire dérivée de tissu adipeux comprenant des cellules souches mésenchymateuses dans la fabrication d'un médicament destiné à être utilisé dans un procédé de diminution du risque qu'un sujet développe de l'ostéoarthrite suite à une chirurgie arthroscopique d'une articulation selon la revendication 6, sachant que le procédé comprend :
- la génération de la suspension cellulaire à partir de tissu adipeux isolé dudit sujet et l'administration de la suspension cellulaire dans les 3 heures à compter de la préparation de ladite suspension cellulaire.

9. Utilisation d'une suspension cellulaire dérivée de tissu adipeux comprenant des cellules souches mésenchymateuses dans la fabrication d'un médicament destiné à être utilisé dans un procédé de diminution du risque qu'un sujet développe de l'ostéoarthrite suite à une chirurgie arthroscopique d'une articulation selon la revendication 6, sachant que le procédé comprend :
- la génération de la suspension cellulaire pour administration à partir de tissu adipeux isolé dudit sujet et l'administration de la suspension cellulaire pendant que le sujet est continuellement sous anesthésie générale.
